(19) **Europäisches Patentamt / European Patent Office / Office européen des brevets**

(11) **EP 2 788 115 B1**

(12) **FASCICULE DE BREVET EUROPEEN**

(45) Date de publication et mention de la délivrance du brevet:
**09.03.2016 Bulletin 2016/10**

(21) Numéro de dépôt: **12797916.9**

(22) Date de dépôt: **06.12.2012**

(51) Int Cl.:
**B01J 19/00** *(2006.01)*    **G09B 23/24** *(2006.01)*
**G01N 25/00** *(2006.01)*

(86) Numéro de dépôt international:
**PCT/EP2012/074699**

(87) Numéro de publication internationale:
**WO 2013/083725 (13.06.2013 Gazette 2013/24)**

(54) **PROCEDE DE DETERMINATION DU MECANISME REACTIONNEL D'UNE REACTION ET DISPOSITIF ASSOCIE**

VERFAHREN ZUR BESTIMMUNG DES REAKTIONSMECHANISMUS EINER REAKTION UND ZUGEHÖRIGE VORRICHTUNG

PROCESS FOR DETERMINING THE REACTION MECHANISM OF A REACTION AND ASSOCIATED DEVICE

(84) Etats contractants désignés:
**AL AT BE BG CH CY CZ DE DK EE ES FI FR GB GR HR HU IE IS IT LI LT LU LV MC MK MT NL NO PL PT RO RS SE SI SK SM TR**

(30) Priorité: **07.12.2011 FR 1161290**

(43) Date de publication de la demande:
**15.10.2014 Bulletin 2014/42**

(73) Titulaires:
* **Centre National de la Recherche Scientifique (CNRS)**
  **75016 Paris (FR)**
* **Université Pierre et Marie Curie (Paris 6)**
  **75005 Paris (FR)**
* **Ecole Normale Supérieure**
  **75230 Paris Cedex 05 (FR)**

(72) Inventeurs:
* **GOSSE, Charlie**
  **F-75014 Paris (FR)**
* **LEMARCHAND, Annie**
  **F-91360 Villemoisson-sur-Orge (FR)**
* **JULLIEN, Ludovic**
  **F-94110 Arcueil (FR)**

* **LE SAUX, Thomas**
  **F-75020 Paris (FR)**
* **ZRELLI, Kais**
  **Riadh Andalous 2035 (TN)**

(74) Mandataire: **Blot, Philippe Robert Emile**
  **Cabinet Lavoix**
  **2, place d'Estienne d'Orves**
  **75441 Paris Cedex 09 (FR)**

(56) Documents cités:
DE-A1-102007 004 624    US-A1- 2005 251 346
US-A1- 2007 043 546

* ZRELLI K ET AL: "Temperature modulated excitation and phase sensitive detection to selectively image DNA sequences", 2011 16TH INTERNATIONAL SOLID-STATE SENSORS, ACTUATORS AND MICROSYSTEMS CONFERENCE (TRANSDUCERS 2011) : BEIJING, CHINA, 5 - 9 JUNE 2011, IEEE, PISCATAWAY, NJ, 5 juin 2011 (2011-06-05), pages 2160-2163, XP031910663, DOI: 10.1109/TRANSDUCERS.2011.5969322 ISBN: 978-1-4577-0157-3

**Description**

La présente invention concerne un procédé de détermination du mécanisme réactionnel d'une réaction, dans laquelle au moins un premier réactif est transformé en au moins un premier produit selon le préambule de la revendication 1.

Un tel procédé s'applique notamment à la détermination du mécanisme chimique de réaction entre des réactifs, en vue d'obtenir des grandeurs thermocinétiques caractéristiques de la réaction.

US 2007/0043546 décrit un procédé de simulation du mécanisme de réactions chimiques.

La bonne connaissance du mécanisme réactionnel permet d'optimiser les conditions dans lesquelles conduire une réaction à l'échelle du laboratoire ou à l'échelle industrielle. Ceci permet par exemple de sélectionner les réactifs et les conditions réactionnelles pour augmenter la vitesse de production d'un produit ou obtenir un rendement amélioré. Ceci permet encore d'optimiser la vitesse de réaction entre molécules dans l'optique de rendre plus efficace un traitement thérapeutique ou plus sensible une méthode de détection.

En variante, cette connaissance permet également de sélectionner ou de cribler des molécules ayant une certaine activité ou susceptibles de s'associer à d'autres molécules, ou à des sites actifs sur des surfaces, afin de déterminer par exemple les applications thérapeutiques de ces molécules ou catalytiques de ces molécules ou de ces surfaces. Cette connaissance enrichit également les modèles de réaction chimique qui sont utilisés dans l'appréhension du monde nous entourant.

La détermination du mécanisme réactionnel d'une réaction est, en général, une opération difficile à réaliser. Elle nécessite l'utilisation de techniques expérimentales compliquées et de protocoles d'études théoriques et pratiques qui peuvent être longs à mettre en oeuvre et coûteux en réactifs.

En général, l'interaction entre deux espèces chimiques A et B est caractérisée par une collection d'actes élémentaires. Le plus souvent, il est possible de réduire cette collection à un nombre limité de réactions qui rendent compte de façon satisfaisante de la dynamique d'un mélange de A et de B à une échelle de temps donnée. On obtient alors un mécanisme réactionnel.

La modélisation par un processus unique de type :

$$aA + bB \underset{k_{-1}}{\overset{k_{+1}}{\longleftrightarrow}} cC \quad (1)$$

est une situation couramment rencontrée en chimie et en biologie. L'interaction entre A et B est alors principalement caractérisée par une stoechiométrie (donnée par les coefficients a, b et c), par des constantes cinétiques $k_{+1}$ et $k_{-1}$ et par une constante thermodynamique $K_1$. A ces trois dernières constantes peuvent se rajouter les énergies d'activation $E_{\pm 1}$ ainsi que l'enthalpie de réaction $\Delta_1 H = E_{+1} - E_{-1}$, le tout formant un ensemble de constantes thermocinétiques associé à la réaction considérée.

Cependant, A et B peuvent interagir selon divers mécanismes (par exemple les mécanismes simples de type A + B = C, ou les mécanismes plus complexes de type 2A + B = C, A + B = 2C, A + B + C =2C, etc) qu'il est parfois difficile de discriminer.

Pour déterminer des constantes cinétiques, on connaît des appareils de mesure dans lesquels les réactifs sont mélangés et une mesure de l'avancement de la réaction au cours du temps est effectuée.

Un premier type d'appareil connu, de type « stopped flow », fonctionne en phase homogène. Diverses solutions contenant les réactifs sont amenées à travers un système de canalisations et d'éléments fluidiques et le mélange formé est recueilli dans une cuvette, dans laquelle sont mesurées différentes propriétés à l'aide de dispositifs de mesure.

De tels appareils ne donnent pas entière satisfaction. Ils nécessitent en effet des volumes de solutions réactives importants, qui ne sont pas toujours disponibles, notamment dans le cas de molécules disponibles en faible quantité ou très coûteuses. Des tentatives de miniaturisation ont bien été entreprises mais c'est alors la gestion du mélange à bas nombre de Reynolds qui devient problématique. De plus, l'utilisation de tels dispositifs est impossible lorsque le milieu réactionnel n'est plus fluide, par exemple lorsque les réactifs forment un gel ou un solide.

Par ailleurs, les mouvements de fluide nécessaires à la mise en oeuvre de cette technique peuvent perturber la réaction, notamment pour des milieux réactionnels complexes et fragiles tels par exemple ceux contenant des objets biologiques.

Un deuxième type d'appareil connu, de type à résonance de plasmon de surface (SPR), fonctionne en phase hétérogène. L'un des réactifs est immobilisé sur une surface et une solution contenant l'autre réactif est injectée dans la chambre fluidique. L'avancement de la réaction est suivi en continu.

Du fait des interactions molécule/surface un tel appareil ne traduit cependant pas les conditions réelles rencontrées lorsque la réaction a lieu en phase homogène, ce qui est la situation la plus classiquement rencontrée.

Par ailleurs, un troisième type d'appareils, de type « T-jump », existe également pour réaliser des mesures cinétiques, en effectuant un saut de température et en déterminant la réponse d'un système à un tel saut de température. Ce type d'appareil effectue une mesure qui n'est pas stationnaire et donc nécessite généralement une perturbation importante du système afin de pouvoir suivre la modification de l'avancement de la réaction avec une résolution suffisante, ce qui peut alors rendre la modélisation des phénomènes délicate. De plus il faudra généralement travailler avec une quantité de réactifs conséquente.

Un quatrième type d'appareil produit une variation périodique d'un paramètre de contrôle expérimental influant sur la cinétique de réaction, puis mesure une grandeur représentative de la concentration d'un réactif à différentes fréquences. Ce type d'appareil n'est pas commercial.

Ainsi, l'article « Lock-in by molecular multiplication » de Braun et al., Appl. Phys. Lett., 83, pages 5554 à 5556 (2003), décrit un procédé de détermination de temps de relaxation chimique ($\tau = 1/(k_{+1} + k_{-1})$ dans le présent cas). Le paramètre de contrôle influant sur la réaction est dans ce cas la température. Les données expérimentales relatives aux concentrations en réactifs obtenues avec différents retards par rapport à l'excitation thermique permettent de mesurer l'amplitude et la phase de la réponse du système chimique, puis d'établir les diagrammes de Bode correspondants. Le temps de relaxation est alors déterminé à partir de ces diagrammes par ajustement numérique.

Pour le calcul des constantes cinétiques, quelle que soit la technique mise en oeuvre, il est généralement connu d'attribuer un mécanisme donné à une réaction, puis de déterminer les constantes cinétiques sur la base du mécanisme attribué. Ceci est notamment vrai pour le quatrième type d'appareil et la démonstration fournie par Braun et al.

Un but de l'invention est d'obtenir un procédé qui permette de déterminer de manière fiable et précise le mécanisme et les paramètres thermocinétiques d'une réaction chimique impliquant au moins un réactif réagissant pour former une espèce produite.

A cet effet, l'invention a pour objet un procédé selon la revendication 1.

Le procédé selon l'invention peut comprendre l'une ou plusieurs des caractéristiques des revendications 2 à 12, prise(s) isolément ou selon toute combinaison techniquement possible.

L'invention a également pour objet un dispositif selon la revendication 13

Le dispositif selon l'invention peut comprendre la caractéristique de la revendication 14.

L'invention sera mieux comprise à la lecture de la description qui va suivre, donnée uniquement à titre d'exemple, et faite en se référant aux dessins annexés, sur lesquels :

- la figure 1 est une vue d'un logigramme décrivant les principales étapes du procédé selon l'invention ;
- la figure 2 est une vue d'un dispositif de mise en oeuvre du procédé selon l'invention ;
- la figure 3 est une vue du comportement théorique d'une première fonction caractéristique d'une grandeur thermocinétique invariante pour un premier mécanisme donné A+B=C, la première fonction étant obtenue pour différents mécanismes supposés de réaction ;

- la figure 4 est une vue analogue à la figure 3, pour une deuxième fonction caractéristique d'une seconde grandeur thermocinétique invariante pour le premier mécanisme A+B=C, la deuxième fonction étant obtenue pour différents mécanismes supposés de réaction ;
- la figure 5 est une vue analogue à la figure 3 pour une troisième fonction caractéristique d'une troisième grandeur thermocinétique invariante pour le premier mécanisme ;
- les figures 6 à 7 illustrent les déterminations expérimentales d'une pluralité de valeurs d'une fonction caractéristique à partir des mesures d'amplitudes d'oscillation, ici au premier ordre en phase et en quadrature de phase, obtenues expérimentalement pour une pluralité de fréquences mesurées ;
- les figures 8 et 9 sont respectivement des vues schématiques en coupe de côté et en coupe de dessus d'un exemple de réacteur permettant de mettre en oeuvre une réaction sous des conditions comprenant une variation d'un paramètre de contrôle influant sur la réaction.

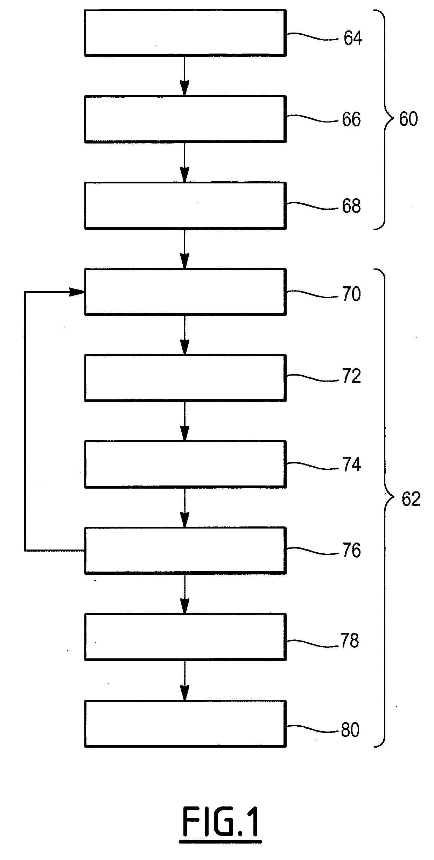

Les principales étapes d'un procédé de détermination selon l'invention sont illustrées par la figure 1.

Le procédé selon l'invention est destiné à déterminer le mécanisme réactionnel d'une réaction dans laquelle au moins une premier réactif A est transformé en au moins un premier produit C, notamment lorsqu'une second réactif B réagit avec A.

Le second réactif B est avantageusement en excès lors de la réaction, de sorte que sa variation de concentration est faible. Ainsi, la concentration du second réactif est avantageusement supérieure à 2 fois la concentration en premier réactif A, et est notamment supérieure à 5 fois la concentration en premier réactif A.

Les espèces A, B et C peuvent être de tout type. Elles sont par exemple sous forme liquide, gazeuse ou solide, pure ou en solution.

Les espèces A, B et C sont par exemple des biomolécules (notamment des acides nucléiques, des protéines, des drogues, des médicaments), des réactifs issus de synthèse organique, inorganique ou minérale, des produits naturels, des complexes métalliques, des catalyseurs, des nanoparticules, voire même des sites d'adsorption à une interface.

La réaction chimique mise en oeuvre est du type :

$$aA + bB \overset{k_{+1}}{\underset{k_{-1}}{\leftrightarrow}} cC \quad (1)$$

Le mécanisme de réaction entre A et B est a priori inconnu avant la mise en oeuvre du procédé. Le procédé selon l'invention a pour objet de le déterminer. En variante, le mécanisme de réaction entre A et B est supposé, et le procédé selon l'invention est destiné à le confirmer.

Le procédé selon l'invention vise aussi, une fois le mécanisme réactionnel connu, à déterminer les constantes thermocinétiques de la réaction par calcul, notamment en vue d'optimiser le déroulement de la réaction ou de gagner en compréhension sur les phénomènes chimiques ou biologiques étudiés. Par ailleurs, la détermination des constantes thermocinétiques donne une indication sur la faisabilité d'une réaction et l'utilité de sa mise en oeuvre dans le cadre d'une production industrielle, ou dans le cadre de l'estimation du potentiel thérapeutique ou catalytique d'une molécule, notamment dans le cadre de criblage d'une série de molécules.

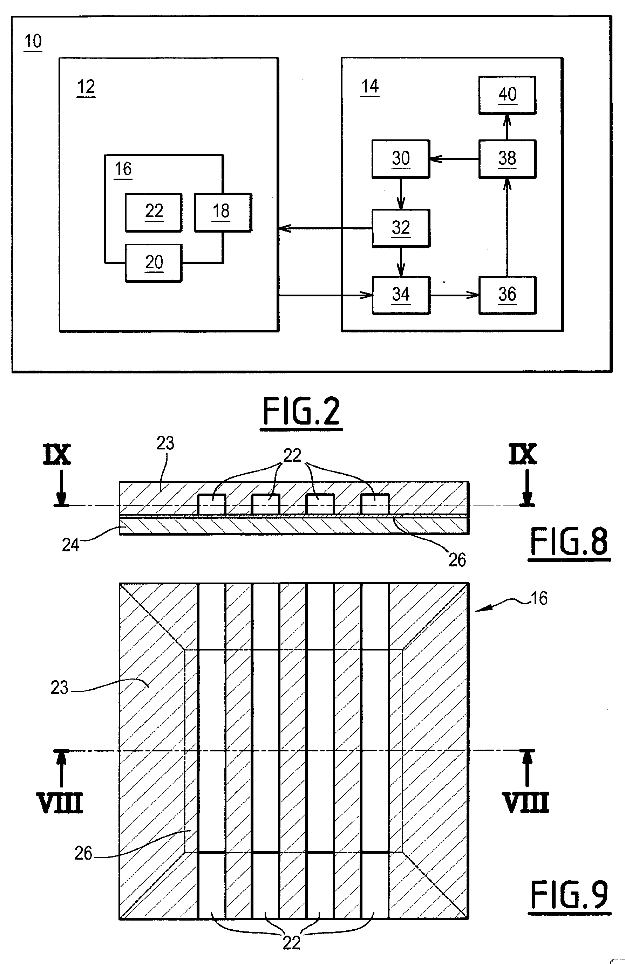

Le procédé selon l'invention est avantageusement mis en oeuvre dans un dispositif 10 représenté sur la Figure 2.

Le dispositif 10 comporte un ensemble 12 de conduite d'une réaction entre les réactifs A, B pour former le produit C. Il comporte un ensemble 14 d'analyse des résultats obtenus lors de chaque réaction menée dans l'ensemble 12.

Comme illustré par la Figure 2, l'ensemble 12 comporte au moins un réacteur 16 dans lequel la transformation d'un premier réactif A en un premier produit C, éventuellement en présence d'un deuxième réactif B en excès, est conduite dans des conditions expérimentales données.

L'ensemble 12 comporte en outre une unité 18 de variation périodique d'une paramètre de contrôle influençant la cinétique de réaction, apte à faire varier périodiquement le paramètre de contrôle à une pluralité de fréquences.

L'ensemble 12 comprend également une unité 20 de mesure permettant de mesurer au moins les amplitudes d'oscillation au premier ordre d'une mesure représentative de la concentration en une espèce A, B, C dans le réacteur 16. Avantageusement, l'unité de mesure 20 est apte à suivre les variations périodiques d'une grandeur représentative de la concentration en premier réactif A dans le milieu réactif.

Avantageusement, la grandeur représentative est proportionnelle à la concentration en premier réactif A dans le milieu réactionnel. Il est ainsi possible de suivre l'avancement de la réaction entre A et B à l'aide de l'unité de mesure 20.

Le réacteur 16 comporte au moins un volume 22 dans laquelle les réactifs A, B sont disposées, et dans laquelle le produit C est obtenu.

Dans l'exemple particulier représenté sur la Figure 8, le réacteur 16 comporte une pluralité de volumes 22 disposés en parallèle les uns avec les autres, pour conduire une pluralité de réactions parallèles sur les mêmes espèces, par exemple à des pulsations $\omega$ d'oscillations différentes, ou sur des réactifs différents.

Les volumes 22 sont par exemple formés par des conduits représentés schématiquement sur la Figure 9, dans lesquels sont disposés le premier réactif A, et le deuxième réactif B, lorsqu'il est présent.

Dans cet exemple, chaque volume 22 est par exemple formé dans une matrice 23 en matière plastique disposée sur une plaque de support 24.

Un exemple d'ensemble réactionnel pouvant être utilisé est décrit dans l'article « Thermal characterisation of a microfluidic cell using the 3 $\Omega$ method » publié dans les « Digest of the Technical Papers of the 14th International Conference on Solid State Sensors, Actuators and Microsystems », 2007, pages U933-U934.

Dans cet exemple, la plaque de support 24 est transparente pour permettre une mesure optique sur le contenu de chaque volume 22.

Chaque volume 22 est de taille comprise entre 1 nm et 100 $\mu$m pour présenter un volume par exemple inférieur à 1 nL.

Plus généralement, il est nécessaire que le volume 22 présente un temps de relaxation thermique inférieur à l'inverse de la pulsation d'oscillation du paramètre de contrôle influant sur la réaction.

Par ailleurs, le volume de réaction 22 n'a pas forcément besoin d'être enclos physiquement, il peut être constitué par une portion du milieu réactif (par exemple une couronne liquide à la surface de colloïdes métalliques chauffés par micro-ondes ou un volume focal dans lequel l'énergie lumineuse fournie par un laser est convertie en chaleur).

Dans une autre variante, le volume 22 forme une goutte de liquide.

Dans un mode de réalisation avantageux, le paramètre de contrôle influant sur la réaction est la température.

En variante, le paramètre de contrôle influant sur la réaction est choisi parmi la pression, la concentration en espèce B ou en un autre composé (proton, sel, agent dénaturant), un flux de particules (photons, ions), un champ (électrique, magnétique).

Dans le cas où le paramètre de contrôle est la température, l'unité 18 de variation périodique comprend un organe 26 de chauffage du contenu de chaque volume 22 et un module 28 de pilotage de chaque organe de chauffage 26 pour engendrer des oscillations sinusoïdales de température de type :

$$T = T_0 \cdot \left[ 1 + \beta \cdot \sin(\omega t) \right] \quad (2)$$

dans le volume 22 autour d'une température moyenne $T_0$ avec une amplitude de chauffage $\beta$, pour une pulsation donnée $\omega$.

Les oscillations de température varient par exemple de 0,1 K à 1 K, pour une gamme de fréquences comprise entre $10^{-3}$ Hz et $10^9$ Hz. Des pulsations de chauffe sont avantageusement comprises notamment entre 5 rad/s et 376 rad/s.

Dans l'exemple représenté sur les Figures 8 et 9, chaque organe de chauffage 26 est formé par une lame chauffante conductrice métallique ou semi-conductrice ou liquide à travers laquelle un courant électrique est injecté.

Cette lame est par exemple formée par une couche de matériau transparent et résistif tel qu'un oxyde d'indium et d'étain. Cette couche a été déposée sur le substrat 24.

Elle présente par exemple une épaisseur inférieure au micromètre, notamment comprise entre 200 nm et 600 nm et est recouverte d'un revêtement en silice pour éviter la manifestation de réactions électrochimiques.

L'organe de chauffage 26 est ainsi apte à provoquer une excitation thermique uniforme sur une surface de la cuve 22.

En variante, le chauffage est produit par l'application d'un faisceau laser, d'un rayonnement électromagnétique micro-ondes, ou par injection de courant électrique dans le milieu réactionnel lorsqu'il est conducteur (provoquant ainsi un chauffage par effet Joule).

Le module de pilotage 28 comporte un générateur électrique, par exemple du type 33220A - 20 MGHz, commercialisé par la société américaine Agilent. Le générateur est apte à produire un courant alternatif sinusoïdal à une pulsation $\omega/2$ égale à la moitié de la pulsation $\omega$ souhaitée pour la température.

L'unité 20 de mesure est apte à mesurer les oscillations d'une grandeur représentative de la concentration d'au moins une espèce A, B, C résultant de la variation périodique du paramètre de contrôle influant sur la réaction, et notamment d'au moins une oscillation de la grandeur représentative de la concentration en réactif A.

L'unité 20 peut aussi déterminer la valeur moyenne de la grandeur représentative de la concentration à la température moyenne $T_0$, mais il est important de noter que toute autre altération non-périodique du signal est très difficile à détecter. Inversement, l'existence d'amplificateurs à détection synchrone rend relativement aisée la caractérisation des réponses du système chimique à l'excitation en température, en amplitude et en phase, pour la première et la seconde harmonique.

Les oscillations de la grandeur représentative sont ensuite reliées aux oscillations de la concentration par une calibration. En particulier, la grandeur représentative est proportionnelle à la concentration.

Avantageusement, l'unité 20 comporte un dispositif de détection optique des variations de concentration d'une espèce A.

Dans un mode de réalisation, l'espèce A dont la concentration est suivie est fluorescente à une longueur d'onde donnée, alors que C l'est moins. Le dispositif de détection optique comporte alors un organe d'émission d'une lumière propre à faire fluorescer l'espèce et un appareil de mesure par fluorescence, tel qu'un microscope à fluorescence.

Un exemple de dispositif de détection est décrit dans l'article « Temperature modulation and quadrature detection for selective titration of two-state exchanging reactants », Analytical Chemistry, 2011, pages 2076 à 2484.

Un tel dispositif est apte à mesurer une grandeur proportionnelle à la concentration en réactif A en fonction du temps, pour en déduire les oscillations périodiques de la concentration en l'espèce A ainsi que le terme stationnaire associé $A^0$.

Avantageusement, le microscope est muni d'une caméra propre à mesurer et à mémoriser, en fonction du temps et de la position dans le volume 22, une intensité de fluorescence.

Les oscillations d'intensité de fluorescence sont ensuite reliées aux oscillations de concentration, par une calibration, comme décrit dans l'article précité.

En variante, d'autres types d'unités de mesure 20 sont utilisées comprenant par exemple des détecteurs tels que des photomultiplicateurs, des photodiodes ou autres composants optoélectroniques, des résonateurs mécaniques, des capteurs plasmoniques, électriques, ou électrochimiques.

La grandeur détectée par l'unité de mesure 20 est donc avantageusement choisie parmi notamment la fluorescence, la masse, l'indice de réfraction, l'intensité d'une raie vibrationnelle, l'absorbance, etc. Cette grandeur est mesurée par un détecteur approprié présent dans l'unité de mesure.

L'ensemble d'analyse 14 est raccordé à l'unité de mesure 20. Il est par exemple formé par une unité comportant un calculateur et une mémoire tel qu'un ordinateur.

Il comporte une unité 30 de choix d'un mécanisme réactionnel supposé de réaction parmi une pluralité de mécanismes réactionnels supposés, une unité 32 chargée de sélectionner au moins une fonction caractéristique d'une grandeur thermocinétique invariante avec la fréquence d'oscillation pour le mécanisme réactionnel supposé par l'unité 30 et chargée de faire réaliser la conduite de réaction par l'unité 12, une unité 34 de calcul d'une pluralité de valeurs de la fonction caractéristique à partir des amplitudes d'oscillation au premier ordre, et éventuellement au second ordre, obtenues expérimentalement dans l'ensemble 12 de conduite d'une réaction.

L'ensemble d'analyse 14 comporte en outre une unité 36 d'analyse des valeurs calculées de la fonction caractéristique pour déterminer si cette fonction est constante, et une unité 38 d'attribution du mécanisme supposé à la réaction lorsque la fonction caractéristique est constante et de réorientation vers la recherche d'un mécanisme alternatif lorsque la fonction n'est pas constante.

L'ensemble d'analyse 14 comporte en outre une unité 40 de calcul d'au moins une constante thermocinétique sur la base du mécanisme réactionnel attribué par l'unité 38.

L'unité 30 comporte une base de données contenant différents mécanismes possibles de réactions tels que les mécanismes suivants :

$$A + B = C, \ A + B = 2\,C, \ 2A + B = C, \ A + B + C = 2C, \ A + B = C = D = A + B, \ \text{etc.}$$

Pour chaque mécanisme présent dans la base de données de l'unité 30, l'unité 32 comporte une base de données de fonctions caractéristiques d'une grandeur thermocinétique invariante avec la fréquence d'oscillation de la grandeur influant sur la réaction.

Ces fonctions représentatives sont notées par la suite $F(\omega)$, $G(\omega)$, $H(\omega)$. Elles seront décrites plus en détail ci-après. Il est cependant important de noter qu'elles se distinguent des fonctions traditionnellement tracées, par exemple l'amplitude et la phase des oscillations au premier ordre (diagramme de Bode), dans la mesure où leur valeur constante permet, en cas de validation du mécanisme supposé, d'obtenir la grandeur thermocinétique caractéristique sans ajustement numérique.

La grandeur thermocinétique invariante peut être une énergie d'activation $E_{\pm1}$ de la réaction ou sa forme réduite :

$\varepsilon_{\pm1} = E_{\pm1}/RT_0$ (3), où R est la constante des gaz parfaits et $T_0$ est la température, ou encore une formule reliant les énergies d'activation ou leurs formes réduites entre elles.

Dans un mode de réalisation, une première fonction caractéristique $F(\omega)$ correspond à la grandeur thermocinétique :

$$\varepsilon_{-1} \cdot (\varepsilon_{-1} - 2) - \varepsilon_{+1} \cdot (\varepsilon_{+1} - 2) \ (4).$$

Une deuxième fonction caractéristique $G(\omega)$ correspond à la grandeur thermocinétique $\tau_1$, qui est le temps de relaxation de la réaction.

Une troisième fonction caractéristique $H(\omega)$ correspond à la grandeur thermocinétique:

$$\frac{\Delta_1 H}{R \cdot T_0} \ (5) \,,$$

où $\Delta_1 H$ est l'enthalpie de réaction

A chaque mécanisme réactionnel supposé présent dans la base de données de l'unité 30 correspond au moins une, avantageusement au moins trois fonctions représentatives $F(\omega)$, $G(\omega)$, $H(\omega)$ dans la base de données de l'unité 32, dont l'expression dépend du mécanisme réactionnel supposé.

Chaque fonction caractéristique $F(\omega)$, $G(\omega)$, $H(\omega)$ présente dans la base de donnée de l'unité 32 est exprimée en fonction d'au moins un paramètre choisi parmi le terme stationnaire $A^0$ des oscillations de concentration en une espèce A, les coefficients $A^{1sin}$, $A^{1cos}$, et éventuellement les coefficients $A^{2sin}$, $A^{2cos}$ qui correspondent respectivement aux amplitudes des oscillations au premier ordre et au deuxième ordre de la concentration en l'espèce A, à la pulsation $\omega$, respectivement en phase et en quadrature de phase.

Chaque fonction caractéristique $F(\omega)$, $G(\omega)$, $H(\omega)$ est en outre avantageusement exprimée en fonction de la pulsation $\omega$, et/ou de la valeur N de la concentration en l'espèce A avant le début de la réaction.

Les fonctions représentatives $F(\omega)$, $G(\omega)$, $H(\omega)$ sont obtenues par la méthode suivante, illustrée pour le mécanisme A + B = C.

Pour chaque mécanisme donné, l'équation de la cinétique chimique du mécanisme réactionnel supposé est établie.

Dans le cas du mécanisme A + B = C précité, avec un excès du second réactif B, cette équation s'écrit :

$$\frac{dA}{dt} = U \cdot A + V \quad (6)$$

où $U = -[k_{+1} \cdot B + k_{-1}]$ (7) et $V = k_{-1} \cdot N$ (8), où N est la quantité de réactif A introduite dans le volume 22.

Puis, une équation de conservation de matière est utilisée pour obtenir la concentration en l'espèce produite C :

$$C = N - A \quad (9)$$

Ensuite, un développement limité de la concentration en l'espèce A est effectué à l'ordre 2 pour obtenir

$$A = A^0 + \beta A^1 + \beta^2 A^2 \quad (10)$$

où :

$$A^1 = A^{1\sin} \cdot \sin(\omega t) + A^{1\cos} \cdot \cos(\omega t)$$
$$A^2 = A^{2\sin} \cdot \sin(2\omega t) + A^{2\cos} \cdot \cos(2\omega t) \quad (11)$$

De la même façon les coefficients U et V peuvent être développés au deuxième ordre pour obtenir :

$$U = U^0 + \beta U^1 + \beta^2 U^2 \quad (12)$$

$$V = V^0 + \beta V^1 + \beta^2 V^2 \quad (13)$$

Les constantes cinétiques sont également développées au deuxième ordre pour obtenir :

$$k_{\pm 1} = k_{\pm 1}^0 + \beta k_{\pm 1}^1 + \beta^2 k_{\pm 1}^2 \quad (13\text{bis}),$$

où

$$k_{\pm 1}^0 = r_{\pm 1} \exp(-\varepsilon_{\pm 1}), \quad k_{\pm 1}^1 = k_{\pm 1}^0 \varepsilon_{\pm 1} \sin(\omega t) \quad (13\text{ter})$$

$$k_{\pm 1}^2 = k_{\pm 1}^0 [\varepsilon_{\pm 1}(\varepsilon_{\pm 1} - 2)] \times [1 - \cos(2\omega t)]/4 \quad (13\text{quater})$$

dans laquelle $r_{\pm 1}$ définit le facteur pré-exponentiel constant figurant dans la loi d'Arrhenius.

Puis, l'équation différentielle est résolue à différents ordres pour exprimer les coefficients $A^0$, $A^{1\sin}$, $A^{1\cos}$, $A^{2\sin}$, $A^{2\cos}$ en fonction des constantes cinétiques.

A l'ordre 0, on obtient :

$$A^0 = \frac{k_{-1}^0 \cdot N}{k_{+1}^0 \cdot B + k_{-1}^0} \quad (14)$$

$$C^0 = N - A^0 \quad (15)$$

A l'ordre 1, la résolution de l'équation différentielle permet de calculer les coefficients $A^{1\sin}$ et $A^{1\cos}$ pour obtenir :

$$A^{1\sin} = -\frac{k_{+1}^0 \cdot B \cdot k_{-1}^0 \cdot N}{(k_{+1}^0 \cdot B + k_{-1}^0)^2 + \omega^2} \cdot \frac{\Delta_1 H}{R \cdot T_0} \quad (16)$$

$$A^{1\cos} = -\frac{\omega}{k_{+1}^0 \cdot B + k_{-1}^0} \cdot A^{1\sin} \quad (17)$$

A l'ordre 2, la résolution de l'équation différentielle donne :

$$A^{2\sin} = \frac{(k_{+1}^0 \cdot B \cdot \varepsilon_{+1} + k_{-1}^0 \cdot \varepsilon_{-1}) \cdot (2\omega \cdot A^{1\sin} - (k_{+1}^0 \cdot B + k_{-1}^0) \cdot A^{1\cos})}{2 \cdot ((2\omega)^2 + (k_{+1}^0 \cdot B + k_{-1}^0)^2)} - $$
$$\frac{\omega \cdot k_{+1}^0 \cdot B \cdot A^0 \cdot (\varepsilon_{-1} \cdot (\varepsilon_{-1} - 2) - \varepsilon_{+1} \cdot (\varepsilon_{+1} - 2))}{2 \cdot ((2\omega)^2 + (k_{+1}^0 \cdot B + k_{-1}^0)^2)} \quad (18)$$

$$A^{2\cos} = \frac{k_{+1}^0 \cdot B + k_{-1}^0}{2\omega} \cdot A^{2\sin} + \frac{k_{+1}^0 \cdot B \varepsilon_{+1} + k_{-1}^0 \cdot \varepsilon_{-1}}{4\omega} \cdot A^{1\cos} \quad (19)$$

Un terme non dépendant du temps peut aussi être trouvé mais sa contribution à la valeur moyenne des oscillations étant pondérée par un coefficient $\beta^2$, elle est négligeable devant $A^0$.
Les formules (14) à (19) obtenues sont directement utilisées pour obtenir :

$$k_{+1}^0 \cdot B = -\omega \frac{N - A^0}{N} \frac{A^{1\sin}}{A^{1\cos}} \quad (20)$$

$$k_{-1}^0 = -\omega \frac{A^0}{N} \frac{A^{1\sin}}{A^{1\cos}} \quad (21)$$

$$\frac{\Delta_1 H}{R \cdot T_0} = -\frac{N A^{1\sin}}{A^0 (N - A^0)} \left(1 + (\frac{A^{1\cos}}{A^{1\sin}})^2\right) \quad (22)$$

La formule (22) correspond à la définition de la troisième fonction caractéristique $H(\omega)$.
La deuxième fonction caractéristique $G(\omega)$ est déduite des équations précédentes par l'expression :

$$\tau_1 = \frac{1}{k_{+1} B + k_{-1}} = -\frac{A^{1\cos}}{\omega \cdot A^{1\sin}} \quad (23)$$

La première fonction caractéristique $F(\omega)$ s'obtient par la formule suivante, à partir notamment des équations (18) et (19) :

$$\varepsilon_{-1} \cdot (\varepsilon_{-1} - 2) - \varepsilon_{+1} \cdot (\varepsilon_{+1} - 2) = \frac{4N}{A^0 \cdot (N - A^0)} \cdot \left( A^{2sin} \cdot \left( \frac{2A^{1cos}}{A^{1sin}} - \frac{A^{1sin}}{A^{1cos}} \right) - 3A^{2cos} \right) \quad (24)$$

La même démarche peut être mise en oeuvre pour obtenir les fonctions représentatives $F(\omega)$, $G(\omega)$, $H(\omega)$ associées aux autres mécanismes supposés A + B = 2 C, 2A + B = C, A + B + C = 2C, A + B = C = D = A + B, etc.

Au moins une fonction caractéristique $F(\omega)$, $G(\omega)$, $H(\omega)$ exprimée en fonction des paramètres précités $A^0$, $A^{1sin}$, $A^{1cos}$, $A^{2sin}$, $A^{2cos}$, N, $\omega$ est donc associée à chaque mécanisme réactionnel supposé pour caractériser ce mécanisme.

Un premier procédé suivant l'invention, pour la détermination du mécanisme réactionnel d'une réaction, va maintenant être décrit, en regard de la Figure 1.

Ce procédé comporte une phase 60 d'expérimentation, conduite dans l'ensemble de mise en oeuvre 12 et une phase 62 d'analyse, conduite dans l'ensemble d'analyse 14 sur la base des données mesurées lors de la phase d'expérimentation 60.

Ainsi, dans la phase d'expérimentation 60, le procédé comporte une étape 64 de conduite d'une réaction de transformation des réactifs A et B en un produit C dans des conditions expérimentales données, dans laquelle un paramètre de contrôle influant sur la réaction est variée périodiquement à une fréquence donnée (correspondant à une pulsation $\omega$), et une étape 66 de balayage d'une pluralité de fréquences données de la variation périodique.

Le procédé comprend en outre une étape 68 de mesure, pour chaque fréquence donnée, d'au moins les amplitudes d'oscillation au premier ordre, en phase et en quadrature de phase, de la grandeur représentative d'une concentration d'au moins une des espèces A, B, C. Ceci permet alors d'obtenir au moins les amplitudes $A^{1sin}$, $A^{1cos}$ de la réponse du système chimique à l'excitation périodique.

La phase d'analyse 62 comporte une étape 70 de choix d'un mécanisme réactionnel supposé de réaction, parmi une pluralité de mécanismes réactionnels supposés, une étape de sélection 72 d'au moins une fonction caractéristique d'une grandeur thermocinétique invariante avec la fréquence d'oscillation pour le mécanisme sélectionné, puis une étape 74 de calcul d'une pluralité de valeurs de la fonction sélectionnée à partir des mesures expérimentales effectuées sur la réponse périodique du système chimique à une excitation périodique appliquée à une pluralité de fréquences.

Le procédé comporte ensuite une étape 76 d'analyse des valeurs calculées pour déterminer si la fonction est constante.

Dans le cas où la fonction est constante, le procédé comporte une étape 78 d'attribution du mécanisme supposé à la réaction conduite expérimentalement, et avantageusement, une étape 80 de calcul de constantes thermocinétiques sur la base du mécanisme réactionnel attribué à la réaction.

Dans le cas où la fonction n'est pas constante, le procédé d'identification de mécanisme revient à l'étape 70 où un nouveau mécanisme réactionnel supposé est sélectionné et où toute la procédure de test est réalisée.

Initialement, lors de l'étape 64, la réaction chimique est mise en oeuvre dans des conditions expérimentales données. Ces conditions expérimentales incluent une concentration de départ en une premier réactif A, l'ajout éventuel d'une deuxième réactif B en excès par rapport au premier réactif A, et la détermination d'une température moyenne $T_0$ autour de laquelle des oscillations de température vont être imposées.

Par « en excès », on entend que la concentration en le deuxième réactif est supérieure à celle du premier réactif A et est notamment supérieure à 2 fois, en particulier supérieure à 3 fois, celle du premier réactif A.

Le premier réactif A, et le deuxième réactif B, lorsqu'il est présent, sont introduits dans un volume 22 puis, une modulation temporelle du paramètre de contrôle influant sur la réaction à une fréquence donnée correspondant à une pulsation $\omega$ est effectuée.

Avantageusement, le paramètre de contrôle influant sur la réaction est la température. La température du volume 22 de réaction est donc modulée autour de la température $T_0$ à la pulsation $\omega$ engendrée suivant l'équation suivante :

$$T = T_0 \cdot [1 + \beta \cdot \sin(\omega t)] \quad (2)$$

Cette modulation temporelle de la température est pilotée par l'unité 18, et est engendrée par exemple par les organes de chauffage 26.

A l'étape 66, une pluralité de fréquences de variation de la fréquence d'oscillation du paramètre de contrôle influant sur la réaction sont balayées soit en série soit en parallèle

Les fréquences balayées, exprimées en terme de pulsation, varient par exemple entre 5 rad/s et 376 rad/s.

Le nombre de fréquences balayées est supérieur à 2, notamment supérieur à au moins 5, voire au moins 10, par exemple compris entre 2 et 100. Plus le nombre de points sera élevé plus grande sera la précision obtenue sur la détermination de la constante thermocinétique effectuée par l'unité 40.

Les mesures doivent être effectuées sur un intervalle tel que les pulsations explorées couvrent au moins deux unités logarithmiques ($\log_{10}$)., De plus cet intervalle doit être centré autour de l'inverse du temps de relaxation chimique supposé pour la réaction étudiée. L'intervalle en question pourra être fixé après une évaluation de $\tau_1$ basée par exemple sur le tracé des diagrammes de Bode.

En variante, l'excitation peut être effectuée par l'unité 18 simultanément à plusieurs pulsations $\omega$, grâce à un générateur de fonctions. L'analyse du signal fourni par l'unité 20 peut elle aussi être réalisée de façon parallélisée, notamment par analyse de Fourier. Le terme balayage n'implique donc pas forcément l'idée d'un protocole expérimental conduit de façon sérielle.

Pour chaque fréquence, la réaction de transformation définie à l'étape 64 est conduite dans les mêmes conditions expérimentales, avec la même température moyenne $T_0$ avantageusement la même amplitude de variation $\beta$, la seule variable étant la pulsation $\omega$.

En variante, l'amplitude de variations $\beta$ change d'une mesure à l'autre. Toutefois, les amplitudes mesurées sont corrigées en fonction de la valeur relative de $\beta$ entre les différentes mesures effectuées aux différentes fréquences.

A l'étape 68, les oscillations de la grandeur représentative de la concentration en au moins une espèce A, B, C, et notamment en le premier réactif A sont mesurées pour chaque fréquence donnée.

En particulier, dans le cas où $\beta$ est inférieur à environ $10^{-2}$, et dans lequel $\beta T_0$ est avantageusement de l'ordre du degré K, les oscillations de concentration en la première espèce A peuvent être développées au deuxième ordre suivant l'équation

$$A = A^0 + \beta\left[A^{1\sin} \cdot \sin(\omega t) + A^{1\cos} \cdot \cos(\omega t)\right] + \beta^2\left[A^{2\sin} \cdot \sin(2\omega t) + A^{2\cos} \cdot \cos(2\omega t)\right] (24)$$

A l'étape 68, l'unité 20 mesure donc les oscillations de la grandeur représentative de la concentration en l'espèce A pour chaque fréquence d'oscillation, et détermine au moins les coefficients $A^{1\sin}$, $A^{1\cos}$, et éventuellement les coefficients $A^{2\sin}$, $A^{2\cos}$ qui correspondent aux amplitudes des oscillations respectivement au premier ordre et au deuxième ordre de la concentration en l'espèce A.

Cette détermination est par exemple effectuée par les méthodes optiques décrites dans l'article de Zrelli et al. cité ci-dessus, sur la base du signal optique mesuré.

Les étapes 66 et 68 sont reproduites pour différentes fréquences de pulsation, par exemple comprises dans une gamme de pulsations entre 5 rad/s et 376 rad/s. Ainsi, il est possible d'obtenir, pour chaque pulsation $\omega$, le terme stationnaire $A^0$, ainsi que les différentes amplitudes $A^{i\sin}$ et $A^{i\cos}$ pour i=1 et éventuellement pour i=2.

Une fois ces données collectées par l'ensemble 12 de mise en oeuvre, lors de la phase 60, la phase d'analyse 62 dans le dispositif 14 est démarrée.

Cette phase comporte initialement une étape de choix 60 d'un mécanisme réactionnel supposé pour la réaction concernée. Avantageusement, le mécanisme supposé est un mécanisme de type A+B=C.

En variante, d'autres mécanismes compris dans l'unité 30 de stockage des mécanismes sont envisageables, tels que ceux décrits plus haut.

Puis, à l'étape 72, au moins une fonction caractéristique $F(\omega)$, $G(\omega)$, $H(\omega)$ d'une grandeur thermocinétique invariante avec la fréquence d'oscillation pour ce mécanisme supposé est sélectionné.

Avantageusement, la grandeur thermocinétique invariante d'une première fonction caractéristique $F(\omega)$ dépend par exemple de l'énergie d'activation $E_{\pm 1}$ de la réaction ou de sa forme réduite :

$$\varepsilon_{\pm 1} = E_{\pm 1}/RT_0 \ (3)$$

ou encore une expression reliant les énergies d'activation entre elles telle que :

$$\varepsilon_{-1} \cdot (\varepsilon_{-1} - 2) - \varepsilon_{+1} \cdot (\varepsilon_{+1} - 2) \ (4).$$

Ainsi, une première fonction caractéristique $F(\omega)$ d'une grandeur thermocinétique est calculée comme décrit précédemment. Selon le mécanisme A+B=C, la fonction $F(\omega)$ s'écrit sous la forme :

$$F(\omega) = \frac{4N}{A^0 \cdot (N-A^0)} \cdot \left( A^{2\sin} \cdot \left( \frac{2A^{1\cos}}{A^{1\sin}} - \frac{A^{1\sin}}{A^{1\cos}} \right) - 3A^{2\cos} \right) \quad (25),$$

où $N$ est la concentration initiale en réactif A et les autres termes sont des amplitudes d'oscillation de la concentration en espèce A au premier ordre et au deuxième ordre, en phase et en quadrature de phase.

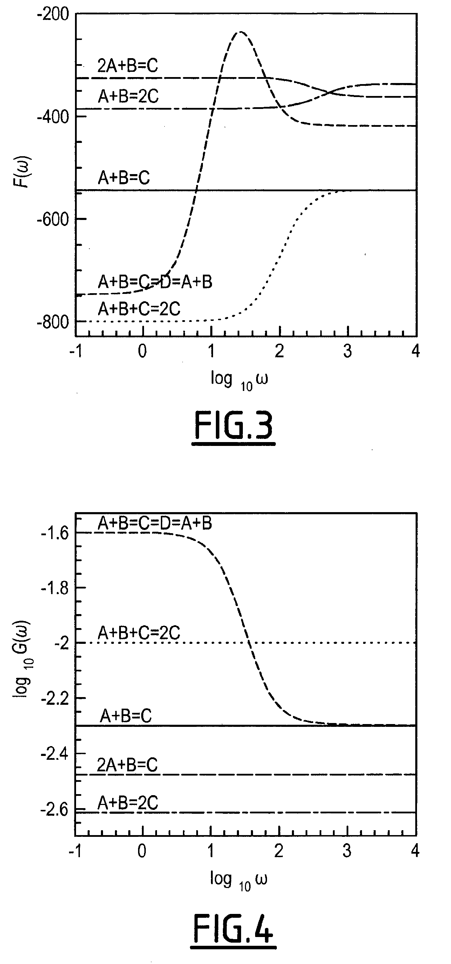

Cette fonction $F(\omega)$ est représentative d'une grandeur thermocinétique invariante avec la fréquence d'oscillation pour le mécanisme A+B=C, comme l'illustre la représentation théorique de la Figure 3.

Au contraire, pour d'autres mécanismes tels que les mécanismes A+B=2C, 2A+B=C, A+B=C=D=A+B ou A+B+C= 2C, la fonction $F(\omega)$ telle que définie par l'équation (25) n'est pas représentative d'une grandeur thermocinétique invariante avec la fréquence et varie en fonction de la fréquence.

Avantageusement, au moins une deuxième fonction caractéristique $G(\omega)$ est calculée, par exemple à partir d'une grandeur thermocinétique invariante avec la fréquence d'oscillation formée par le temps de relaxation $\tau_1$.

Comme décrit précédemment pour le mécanisme A+B=C, la fonction $G(\omega)$ est alors de la forme :

$$G(\omega) = -\frac{A^{1\cos}}{\omega \cdot A^{1\sin}} \quad (26).$$

Comme visible sur la Figure 3, la fonction $G(\omega)$ est en théorie constante pour certains mécanismes réactionnels (A+B = C, A+B+C = 2C, 2A+B = C, A+B = 2C), alors qu'elle varie pour d'autres mécanismes réactionnels (A+B+C=C=D=A+B).

Encore avantageusement, une troisième fonction caractéristique $H(\omega)$ d'une grandeur thermocinétique invariante avec la fréquence d'oscillation pour le mécanisme réactionnel supposé est sélectionnée, sur la base du rapport entre l'enthalpie de réaction $\Delta_1 H$ et le produit $R \cdot T_0$ de la constante des gaz parfaits R par la température moyenne $T_0$.

Selon le mécanisme A+B=C, cette fonction $H(\omega)$ s'écrit comme suit :

$$H(\omega) = -\frac{NA^{1\sin}}{A^0(N-A^0)}\left( 1 + (\frac{A^{1\cos}}{A^{1\sin}})^2 \right) \quad (27)$$

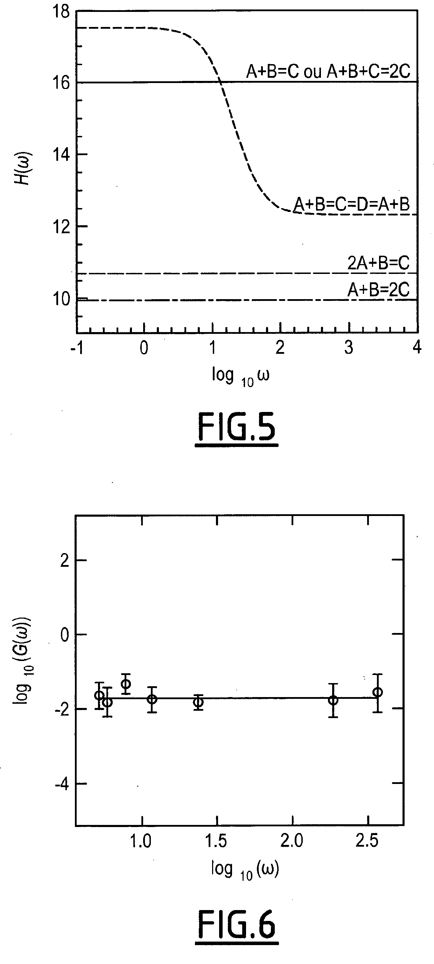

Comme le montre la figure 5, la fonction $H(\omega)$ est en théorie constante pour différents mécanismes (A+B=2C, 2A+B=C, A+B+C=2C), alors qu'elle ne l'est pas pour le mécanisme A+B=C=D=A+B.

A l'étape 74, les mesures du terme stationnaire $A^0$ et d'amplitudes d'oscillation $A^{1\sin}$, $A^{1\cos}$, $A^{2\sin}$, $A^{2\cos}$ déterminées expérimentalement au premier ordre et éventuellement au deuxième ordre, sont utilisées pour calculer une pluralité de valeurs de chaque fonction caractéristique sélectionnée à l'étape 72 à la pluralité de fréquences mesurées de variations périodiques de la grandeur influant sur la réaction.

Ainsi pour chaque fréquence mesurée expérimentalement, les valeurs des coefficients $A^0$, $A^{1\sin}$, $A^{1\cos}$, $A^{2\sin}$, $A^{2\cos}$ déterminés expérimentalement pour cette fréquence sont introduits dans les équations (25) à (27) ci-dessus. Ceci permet de calculer une valeur de la fonction caractéristique $F(\omega)$, $G(\omega)$ et $H(\omega)$ associée à chaque fréquence.

Une pluralité de valeurs de chaque fonction $F(\omega)$, $G(\omega)$ et $H(\omega)$ sont calculées à partir des mesures d'amplitudes d'oscillation au premier ordre et éventuellement au deuxième ordre obtenues expérimentalement, pour une pluralité de fréquences.

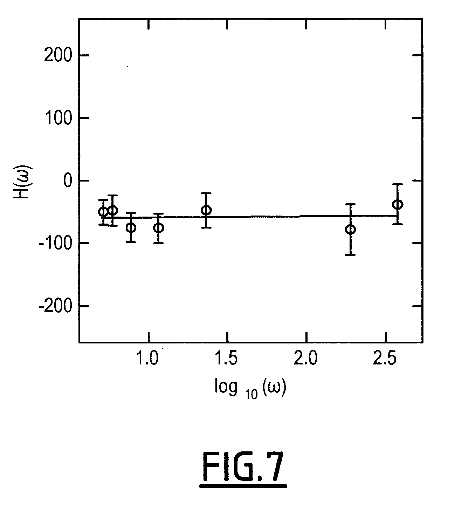

Des exemples de courbes expérimentales de valeurs des fonctions $G(\omega)$ et $H(\omega)$ sont données sur les figures 6 et 7.

Puis, à l'étape 74, les valeurs calculées de chaque fonction $F(\omega)$, $G(\omega)$ et $H(\omega)$ sont analysées pour déterminer si la fonction $F(\omega)$, $G(\omega)$ ou $H(\omega)$ est constante.

Par exemple, l'écart type empirique corrigé calculé à partir de la pluralité des valeurs de la fonction doit être inférieur à la moyenne des incertitudes expérimentales sur les valeurs de la fonction, mesurées ou estimées, pour chacune des pulsations.

Ainsi, la valeur de la fonction caractéristique F, G, H est déterminée à chaque fréquence de mesure, on obtient un ensemble de n valeurs $F(\omega_i)$, $G(\omega_i)$, $H(\omega_i)$. L'incertitude sur chaque valeur est mesurée ou estimée, on obtient un ensemble de $\sigma_F(\omega_i)$, $\sigma_G(\omega_i)$, $\sigma H(\omega_i)$.

Puis, la moyenne des valeurs de la fonction caractéristique obtenues pour les différentes fréquences est calculée : $<F> = 1/n \sum_i F(\omega_i)$, $<G> = 1/n \sum_i G(\omega_i)$, $<H> = 1/n \sum_i H(\omega_i)$. Ceci permet alors de déterminer l'écart type empirique corrigé pour l'ensemble de données constitué par les valeurs de la fonction sur la pluralité des fréquences auxquelles ont était effectuée les mesures : $E_F = \sqrt{1/(n-1)\Sigma_i [F(\omega_i)- <F>]^2}$, $E_G = \sqrt{1/(n-1) \Sigma_i [G(\omega_i)-<G>]^2}$, $E_H = \sqrt{1/(n-1) \Sigma_i [H(\omega_i)- <H>]^2}$.

Enfin, la moyenne des incertitudes est calculée : $<\sigma_F> = 1/n \Sigma_i \sigma_F (\omega_i)$, $<\sigma_G> = 1/n \Sigma_i \sigma_G (\omega_i)$, $<\sigma_H> = 1/n \Sigma_i \sigma_H(\omega_i)$.

Pour que la fonction soit déclarée constante, il suffit que $E_F \leq <\sigma_F>$, $E_G \leq <\sigma_G>$, $E_H \leq <\sigma_H>$.

Dans un autre exemple particulier, la fonction $F(\omega)$, $G(\omega)$, ou $H(\omega)$ est considérée comme constante, si l'écart type empirique corrigé des valeurs déterminées pour la fonction est inférieur à un seuil donné, par exemple inférieur à 30 % de la moyenne.

A l'étape 78, si chaque fonction $F(\omega)$, $G(\omega)$, $H(\omega)$ caractéristique supposée être constante pour le mécanisme donné est considérée comme constante à l'étape 74, le mécanisme supposé est attribué à la réaction.

En particulier, lorsque la fonction $F(\omega)$ est constante suivant l'étape 76, le mécanisme réactionnel A+B=C est attribué à la réaction, puisque ce mécanisme est le seul pouvant donner une valeur de $F(\omega)$ constante.

Lorsqu'un mécanisme est attribué à la réaction, les fonctions $G(\omega)$ et $H(\omega)$ peuvent confirmer l'attribution du mécanisme en question.

Au contraire, si une fonction $F(\omega)$ supposée être constante pour le mécanisme supposé ne l'est pas à l'issue de l'étape 76, le procédé peut comprendre un retour à l'étape 70 pour choisir un autre mécanisme réactionnel supposé de réaction, et sélectionner de nouvelles expressions de fonctions représentatives de grandeurs thermocinétiques invariantes avec la fréquence d'oscillation pour ce nouveau mécanisme réactionnel supposé.

L'étape 74 est alors répétée pour déterminer si la ou les nouvelles fonctions caractéristiques sont effectivement constantes.

Dans une variante, lorsque la fonction caractéristique $G(\omega)$, $H(\omega)$ ne permet pas de discriminer entre plusieurs mécanismes supposés, l'étape d'attribution peut comprendre une étape dans laquelle des valeurs particulières d'une fonction caractéristique auxiliaire $I(\omega)$ non constante pour ce mécanisme sont calculées, pour déterminer un critère discriminant entre plusieurs fonctions représentatives sur la base de ce calcul.

En particulier, lorsque la fonction caractéristique $F(\omega)$ n'est pas constante mais que les fonctions $G(\omega)$ et $H(\omega)$ sont constantes, le mécanisme peut être du type A+B=2C, 2A+B=C, ou A+B+C=2C. La fonction $I(\omega)$ est par exemple calculée comme la différence entre la valeur $F(\omega)$ à une pulsation $\omega \gg 1/\tau_1$ et la valeur asymptotique $F(0)$ à fréquence nulle.

Si cette différence est négative, le mécanisme 2A+B=C peut être attribué alors que si cette différence est positive, un choix devra être fait entre le mécanisme A+B=2C et le mécanisme A+B+C=2C.

Une fois le mécanisme expérimental attribué à l'étape 78, l'étape 80 de calcul est mise en oeuvre.

Cette étape permet de calculer directement, à partir des amplitudes d'oscillation expérimentales $A^{1sin}$, $A^{1cos}$, $A^{2sin}$, $A^{2cos}$, les grandeurs thermocinétiques telles que les énergies d'activation, $E_{+1}$ et $E_{-1}$, respectivement associées à la formation et à la dissociation du produit, l'enthalpie de réaction $\Delta_1 H$ et le temps de relaxation $\tau_1$.

Par exemple, pour le mécanisme A+B=C, les différentes constantes peuvent être calculées par les équations (22) à (24) précédemment citées.

Il est ainsi possible d'estimer, de manière simple et précise, les paramètres thermocinétiques. Une telle détermination permet notamment d'optimiser les procédés de réaction, ou de cribler des molécules susceptibles de présenter une activité intéressante, de type thérapeutique ou catalytique par exemple.

Le procédé selon l'invention peut être mis en oeuvre avantageusement avec des quantités faibles de réactif, ce qui diminue son coût. En outre, un seul échantillon est nécessaire pour la variation de la fréquence de modulation, ce qui simplifie le procédé.

Dans un mode de réalisation particulier, l'unité 14 comporte un moyen d'affichage (non représenté) du mécanisme de réaction déterminé, ainsi que des grandeurs thermocinétiques calculées.

## Revendications

1. Procédé de détermination du mécanisme réactionnel d'une réaction dans laquelle au moins une premier réactif (A) est transformé en au moins un premier produit (C), comportant les étapes suivantes :

   (a1) conduite d'une réaction de transformation du premier réactif (A) en premier produit (C) dans des conditions expérimentales données, la réaction comprenant la variation périodique à une fréquence donnée d'un paramètre de contrôle (T) influant sur la réaction ;
   (a2) balayage d'une pluralité de fréquences données de variations périodiques du paramètre de contrôle (T) influant sur la réaction ;
   (a3) pour chaque fréquence étudiée, mesure d'une grandeur représentative de la concentration d'au moins une des espèces sous l'effet de la variation périodique, pour déterminer au moins les amplitudes d'oscillation ($A^{1sin}$, $A^{1cos}$) au premier ordre de la concentration ,

**caractérisé en ce que** le procédé comporte en outre les étapes suivantes :

(a4) choix d'un mécanisme réactionnel supposé de réaction ;

(a5) sélection d'au moins une première fonction (F($\omega$)) caractéristique d'une grandeur thermocinétique invariante avec la fréquence d'oscillation pour ledit mécanisme réactionnel supposé, la première fonction caractéristique (F($\omega$)) étant calculée au moins à partir des amplitudes d'oscillation ($A^{1sin}$, $A^{1cos}$) au premier ordre de la concentration (A) d'au moins ladite espèce ;

(a6) calcul d'une pluralité de valeurs de la première fonction caractéristique (F($\omega$)) à partir des amplitudes d'oscillation au premier ordre ($A^{1sin}$, $A^{1cos}$) obtenues expérimentalement pour la pluralité de fréquences de l'excitation périodique décrite à l'étape (a2) ;

(a7) analyse des valeurs calculées de la première fonction caractéristique (F($\omega$)) pour déterminer si la première fonction caractéristique (F($\omega$)) est constante sur la base des valeurs calculées ;

(a8) lorsque la première fonction caractéristique (F($\omega$)) est constante, attribution du mécanisme supposé à ladite réaction.

2. Procédé selon la revendication 1, **caractérisé en ce que** le paramètre de contrôle (T) influant sur la réaction est la température, l'étape (a1) comportant la variation périodique de la température à la fréquence donnée, l'étape (a2) comportant le balayage d'une pluralité de fréquences de variation de la température.

3. Procédé selon la revendication 1 ou la revendication 2, **caractérisé en ce qu'**il comporte pour chaque fréquence, la détermination des amplitudes d'oscillation ($A^{2sin}$, $A^{2cos}$) au deuxième ordre de la concentration en au moins une des espèces, la première fonction (F($\omega$) caractéristique sélectionnée à l'étape (a5) étant calculée sur la base des amplitudes d'oscillation au premier ordre ($A^{1sin}$, $A^{1cos}$), et sur la base des amplitudes d'oscillations au deuxième ordre ($A^{2sin}$, $A^{2cos}$).

4. Procédé selon l'une quelconque des revendications précédentes, **caractérisé en ce que** la première fonction caractéristique (F($\omega$)) est calculée sur la base d'une forme réduite ($\varepsilon_{\pm 1}$) du rapport des énergies d'activation sur la constante des gaz parfaits multipliée par la température moyenne de l'échantillon.

5. Procédé selon l'une quelconque des revendications précédentes, **caractérisé en ce qu'**il comporte la sélection d'au moins une deuxième fonction caractéristique (G($\omega$)) ; (H($\omega$)) d'une deuxième grandeur thermocinétique invariante avec la fréquence d'oscillation pour ledit mécanisme supposé, la deuxième fonction caractéristique (G($\omega$)) ; (H(($\omega$)) étant calculée à partir d'au moins les amplitudes d'oscillation ($A^{1sin}$, $A^{1cos}$) au premier ordre de la concentration (A) d'au moins ladite espèce, le procédé comportant la détermination d'une pluralité de valeurs de ladite deuxième fonction caractéristique (G($\omega$)) ; (H($\omega$) à partir des mesures d'amplitudes d'oscillation au premier ordre obtenues à l'étape (a3) pour une pluralité de fréquences de l'excitation périodique et l'analyse des valeurs calculées de la deuxième fonction caractéristique (G($\omega$)) ; (H(($\omega$)) pour déterminer si la deuxième fonction caractéristique (G($\omega$)) ; (H($\omega$) est constante.

6. Procédé selon l'une quelconque des revendications précédentes, **caractérisé en ce que** la deuxième fonction caractéristique (G($\omega$)) est calculée sur la base du temps de relaxation ($\tau_1$) du mécanisme réactionnel supposé.

7. Procédé selon l'une quelconque des revendications précédentes, **caractérisé en ce que** la deuxième fonction caractéristique (H($\omega$)) est calculée en fonction de l'enthalpie de réaction ($\Delta H_1$) du mécanisme réactionnel supposé.

8. Procédé selon l'une quelconque des revendications précédentes, **caractérisé en ce qu'**il comporte la sélection d'une fonction caractéristique auxiliaire (I($\omega$)) non constante pour le mécanisme réactionnel supposé, la fonction caractéristique auxiliaire (I($\omega$)) présentant un signe constant quelque soit la fréquence d'oscillation pour le mécanisme réactionnel supposé, le procédé comportant la détermination d'une pluralité de valeurs de la fonction caractéristique auxiliaire (I($\omega$)) à partir des mesures d'amplitude d'oscillation au premier ordre obtenues à l'étape (a3), et l'analyse du signe de ces valeurs pour déterminer si le signe est constant.

9. Procédé selon l'une quelconque des revendications précédentes, **caractérisé en ce que** la première fonction caractéristique (F($\omega$)) est exprimée en fonction d'au moins un paramètre choisi parmi le terme stationnaire $A^0$ des variations de concentration en une espèce A, les coefficients $A^{1sin}$, $A^{cos}$, et éventuellement les coefficients $A^{2sin}$, $A^{2cos}$ qui correspondent aux amplitudes des oscillations respectivement au premier ordre et au deuxième ordre de la concentration en cette espèce A, la pulsation $\omega$, et/ou la valeur N de la concentration en cette espèce A avant le début de la réaction.

**10.** Procédé selon l'une quelconque des revendications précédentes, **caractérisé en ce que** le mécanisme supposé est choisi parmi un mécanisme de type A + B = C ; A + B = 2 C, 2A + B = C, A + B + C = 2C, A + B = C = D = A + B.

**11.** Procédé selon l'une quelconque des revendications précédentes, **caractérisé en ce qu'**il comporte une étape de calcul d'au moins une constante thermocinétique ($\tau_1$, $\Delta_1$H), sur la base d'équations représentatives de cette constante pour le mécanisme attribué à l'étape (a8), les équations représentatives dépendant au moins des amplitudes mesurées au premier ordre ($A^{1sin}$, $A^{1cos}$).

**12.** Procédé selon l'une quelconque des revendications précédentes, **caractérisé en ce que** lorsque la première fonction caractéristique ($F(\omega)$) n'est pas constante à l'étape (a7), le procédé comporte le choix d'un autre mécanisme réactionnel supposé de réaction et la répétition des étapes (a5) à (a8) pour l'autre mécanisme réactionnel supposé.

**13.** Dispositif (10) de détermination du mécanisme réactionnel d'une réaction dans laquelle au moins un premier réactif (A) est transformée en au moins un premier produit (C), comportant :

- un ensemble (12) de conduite d'une réaction de transformation du premier réactif (A) en premier produit (C) dans des conditions expérimentales données, l'ensemble de conduite (12) comprenant une unité (18) de variation périodique d'un paramètre de contrôle (T) influant sur la réaction à une fréquence donnée ;
- des moyens (28) de balayage d'une pluralité de fréquences données de variations périodiques du paramètre de contrôle influant sur la réaction ;
- une unité (20) de mesure, pour chaque fréquence, d'une grandeur représentative de la concentration d'au moins une des espèces (A) sous l'effet de la variation périodique, propre à déterminer au moins les amplitudes d'oscillation au premier ordre ($A^{1sin}$, $A^{1cos}$) de la concentration en cette espèce.

**caractérisé en ce que** le dispositif (10) comporte en outre un ensemble d'analyse (14) comprenant :

- une unité (30) de choix d'un mécanisme réactionnel supposé de réaction ;
- une unité (32) de sélection d'au moins une première fonction caractéristique ($F(\omega)$) d'une grandeur thermocinétique invariante avec la fréquence d'oscillation pour ledit mécanisme réactionnel supposé, la première fonction caractéristique ($F(\omega)$) étant calculée au moins à partir des amplitudes d'oscillation ($A^{1sin}$, $A^{1cos}$) au premier ordre de la concentration d'au moins ladite espèce (A);
- une unité (34) de calcul d'une pluralité de valeurs de la première fonction caractéristique ($F(\omega)$) à partir des mesures d'amplitudes d'oscillation ($A^{1sin}$, $A^{1cos}$) au premier ordre obtenues expérimentalement pour la pluralité de fréquences de l'excitation périodique générées à l'aide de l'unité (28) ;
- une unité (36) d'analyse des valeurs calculées de la première fonction caractéristique ($F(\omega)$) pour déterminer si la première fonction caractéristique ($F(\omega)$) est constante sur la base des valeurs calculées ;
- une unité (38) d'attribution du mécanisme supposé à ladite réaction lorsque la première fonction caractéristique ($F(\omega)$) est constante.

**14.** Dispositif selon la revendication 13, **caractérisé en ce qu'**il comporte une unité de calcul d'une constante thermocinétique sur la base d'équations représentatives de cette constante pour le mécanisme attribué par l'unité d'attribution (38), les équations représentatives dépendant au moins des amplitudes mesurées au premier ordre ($A^{1sin}$, $A^{1cos}$).

**Patentansprüche**

**1.** Verfahren zur Bestimmung des Reaktionsmechanismus einer Reaktion, in welcher mindestens ein erstes Reagenz (A) in mindestens ein erstes Produkt (C) umgesetzt wird, aufweisend die folgenden Schritte:

(a1) Durchführen einer Reaktion des Umsetzens des ersten Reagenzes (A) in ein erstes Produkt (C) unter gegebenen experimentellen Bedingungen, wobei die Reaktion die periodische Variation eines Steuerparameters (T) bei einer gegebenen Frequenz aufweist, welcher die Reaktion beeinflusst;
(a2) Abtasten einer Mehrzahl von gegebenen Frequenzen von periodischen Variationen des Steuerparameters (T), welcher die Reaktion beeinflusst;
(a3) für jede betrachtete Frequenz Messen einer repräsentativen Größe der Konzentration mindestens einer der Spezies unter der Wirkung der periodischen Variation, um mindestens die Schwingungsamplituden ($A^{1sin}$, $A^{1cos}$) erster Ordnung der Konzentration zu bestimmen,

**dadurch gekennzeichnet, dass** das Verfahren zusätzlich die folgenden Schritte aufweist:

(a4) Auswählen eines angenommenen Reaktionsmechanismus der Reaktion;

(a5) Auswählen mindestens einer ersten Funktion ($F(\omega)$), welche charakteristisch für eine thermokinetische Größe ist, welche invariant unter der Schwingfrequenz des angenommenen Reaktionsmechanismus ist, wobei die erste charakteristische Funktion ($F(\omega)$) mindestens ausgehend von den Schwingungsamplituden ($A^{1sin}$, $A^{1cos}$) erster Ordnung der Konzentration (A) mindestens der Spezies berechnet wird;

(a6) Berechnen einer Mehrzahl von Werten der ersten charakteristischen Funktion ($F(\omega)$) ausgehend von den Schwingungsamplituden erster Ordnung ($A^{1sin}$, $A^{1cos}$), welche experimentell für die Mehrzahl von Frequenzen der in Schritt (a2) beschriebenen periodischen Anregung erhalten sind;

(a7) Analyse der berechneten Werte der ersten charakteristischen Funktion ($F(\omega)$), um zu bestimmen, ob die erste charakteristische Funktion ($F(\omega)$) konstant auf der Basis der berechneten Werte ist;

(a8) wenn die erste charakteristische Funktion ($F(\omega)$) konstant ist, Zuordnen des angenommenen Mechanismus zu der Reaktion.

2. Verfahren nach Anspruch 1, **dadurch gekennzeichnet, dass** der Steuerparameter (T), welcher der Reaktion beeinflusst, die Temperatur ist, wobei der Schritt (a1) die periodische Variation der Temperatur bei der angegeben Frequenz aufweist, wobei der Schritt (a2) die Abtastung einer Mehrzahl von Frequenzen von Temperaturvariationen aufweist.

3. Verfahren nach Anspruch 1 oder Anspruch 2, **dadurch gekennzeichnet, dass** es für jede Frequenz die Bestimmung der Schwingungsamplituden ($A^{2sin}$, $A^{2cos}$) zweiter Ordnung der Konzentration mindestens einer der Spezies aufweist, wobei die erste charakteristische in Schritt (a5) ausgewählte Funktion ($F(\omega)$) auf Basis der Schwingungsamplituden erster Ordnung ($A^{1sin}$, $A^{1cos}$) und auf Basis der Schwingungsamplituden zweiter Ordnung ($A^{2sin}$, $A^{2cos}$) berechnet wird.

4. Verfahren nach einem der vorhergehenden Ansprüche, **dadurch gekennzeichnet, dass** die erste charakteristische Funktion ($F(\omega)$) auf Basis einer reduzierten Form ($\varepsilon_{\pm 1}$) des Verhältnisses zwischen den Aktivierungsenergien und der Konstante der idealen Gase, welche mit der Durchschnittstemperatur der Probe multipliziert ist, berechnet wird.

5. Verfahren nach einem der vorhergehenden Ansprüche, **dadurch gekennzeichnet, dass** es die Auswahl mindestens einer zweiten charakteristischen Funktion ($G(\omega)$; $H(\omega)$) einer zweiten thermokinetischen Größe aufweist, welche unter der Schwingfrequenz für den angenommenen Mechanismus invariant ist, wobei die zweite charakteristische Funktion ($G(\omega)$; $H(\omega)$) ausgehend von den Schwingungsamplituden ($A^{1sin}$, $A^{1cos}$) erster Ordnung der Konzentration (A) mindestens der Spezies berechnet wird, wobei das Verfahren die Bestimmung einer Mehrzahl von Werten der zweiten charakteristischen Funktion ($G(\omega)$; $H(\omega)$) ausgehend von den Messungen von in Schritt (a3) erhaltenen Schwingungsamplituden erster Ordnung für eine Mehrzahl von Frequenzen der periodischen Anregung und die Analyse der berechneten Werte der zweiten charakteristischen Funktion ($G(\omega)$; $H(\omega)$) aufweist, um zu bestimmen, ob die zweite charakteristische Funktion ($G(\omega)$; $H(\omega)$) konstant ist.

6. Verfahren nach einem der vorhergehenden Ansprüche, **dadurch gekennzeichnet, dass** die zweite charakteristische Funktion ($G(\omega)$) auf Basis der Relaxationszeit ($\tau_1$) des angenommenen Reaktionsmechanismus berechnet wird.

7. Verfahren nach einem der vorhergehenden Ansprüche, **dadurch gekennzeichnet, dass** die zweite charakteristische Funktion ($H(\omega)$) auf Basis der Reaktionsenthalpie ($\Delta H_1$) des angenommenen Reaktionsmechanismus berechnet wird.

8. Verfahren nach einem der vorhergehenden Ansprüche, **dadurch gekennzeichnet, dass** es die Auswahl einer charakteristischen Hilfsfunktion ($I(\omega)$) aufweist, welche für den angenommenen Reaktionsmechanismus nicht konstant ist, wobei die charakteristische Hilfsfunktion ($I(\omega)$) ein konstantes Vorzeichen unabhängig von der Schwingfrequenz für den angenommenen Reaktionsmechanismus aufweist, wobei das Verfahren die Bestimmung einer Mehrzahl von Werten der charakteristischen Hilfsfunktion ($I(\omega)$) ausgehend von den Bemessungen der in Schritt (a3) erhaltenen Schwingungsamplituden erster Ordnung aufweist, und die Analyse des Vorzeichens dieser Werte, um zu bestimmen, ob das Vorzeichen konstant ist.

9. Verfahren nach einem der vorhergehenden Ansprüche, **dadurch gekennzeichnet, dass** die erste charakteristische Funktion ($F(\omega)$) in Abhängigkeit von mindestens einem Parameter ausgedrückt ist, ausgewählt aus dem stationären Element $A^0$ der Konzentrationsvariationen einer Spezies A, den Koeffizienten $A^{1sin}$, $A^{1cos}$, und gegebenenfalls den

EP 2 788 115 B1

Koeffizienten $A^{2sin}$, $A^{2cos}$, welche den Schwingungsamplituden erster Ordnung bzw. zweiter Ordnung der Konzentration dieser Spezies A entsprechen, der Winkelfrequenz $\omega$, und/oder dem Wert N der Konzentration der Spezies A vor dem Reaktionsbeginn.

**10.** Verfahren nach einem der vorhergehenden Ansprüche, **dadurch gekennzeichnet, dass** den angenommenen Mechanismus aus einem Mechanismus des Typs A + B = C ; A + B = 2C, 2A + B = C, A+ B + C = 2C, A + B = C = D = A +B ausgewählt ist.

**11.** Verfahren nach einem der vorhergehenden Ansprüche, **dadurch gekennzeichnet, dass** es einen Schritt des Berechnens mindestens einer thermokinetischen Konstante ($\tau_1$, $\Delta_1 H$), auf der Basis repräsentativer Gleichungen dieser Konstante für den in Schritt (a8) zugeordneten Mechanismus aufweist, wobei die repräsentativen Gleichungen von mindestens den gemessenen Amplituden erster Ordnung ($A^{1sin}$, $A^{1cos}$) abhängen.

**12.** Verfahren nach einem der vorhergehenden Ansprüche, **dadurch gekennzeichnet, dass**, wenn die erste charakteristische Funktion ($F(\omega)$) in Schritt (a7) nicht konstant ist, das Verfahren die Auswahl eines anderen angenommenen Reaktionsmechanismus von Reaktion und die Wiederholung der Schritte (a5) bis (a8) für den anderen angenommenen Reaktionsmechanismus aufweist.

**13.** Vorrichtung (10) zur Bestimmung des Reaktionsmechanismus einer Reaktion, in der mindestens ein erstes Reagenz (A) in mindestens ein erstes Produkt (C) umgesetzt wird, aufweisend:

- eine Anordnung (12) zum Durchführen einer Reaktion des Umsetzens des ersten Reagenzes (A) in ein erstes Produkt (C) unter gegebenen experimentellen Bedingungen, wobei die Anordnung (12) eine Einheit (18) der periodischen Variation eines Steuerparameters (T) aufweist, welcher die Reaktion bei einer gegebenen Frequenz beeinflusst;
- Mittel (28) zum Abtasten einer Mehrzahl von gegebenen Frequenzen von periodischen Variationen des Steuerparameters (T), welcher die Reaktion beeinflusst;
- eine Einheit (20) zum Messen einer repräsentativen Größe der Konzentration mindestens einer der Spezies für jede Frequenz unter der Wirkung der periodischen Variation, welche geeignet ist, mindestens die Schwingungsamplituden ($A^{1sin}$, $A^{1cos}$) erster Ordnung der Konzentration dieser Spezies zu bestimmen,

**dadurch gekennzeichnet, dass** die Vorrichtung (10) zusätzlich eine Analyseeinheit (14) aufweist, aufweisend:

- eine Einheit (30) zum Auswählen eines angenommenen Reaktionsmechanismus der Reaktion;
- eine Einheit (32) zum Auswählen mindestens einer ersten charakteristischen Funktion ($F(\omega)$) einer thermokinetischen Größe, welche invariant unter der Schwingfrequenz des angenommenen Reaktionsmechanismus ist, wobei die erste charakteristische Funktion ($F(\omega)$) mindestens ausgehend von den Schwingungsamplituden ($A^{1sin}$, $A^{1cos}$) erster Ordnung der Konzentration mindestens der Spezies (A) berechnet wird;
- eine Einheit (34) zum Berechnen einer Mehrzahl von Werten der ersten charakteristischen Funktion ($F(\omega)$) ausgehend von den Schwingungsamplituden ($A^{1sin}$, $A^{1cos}$) erster Ordnung, welche experimentell für die Mehrzahl von Frequenzen der in Schritt (a2) mittels der Einheit (28) erzeugten periodischen Anregung erhalten werden;
- eine Einheit (36) zur Analyse der berechneten Werte der ersten charakteristischen Funktion ($F(\omega)$), um zu bestimmen, ob die erste charakteristische Funktion ($F(\omega)$) konstant auf der Basis der berechneten Werte ist;
- eine Einheit (38) zum Zuordnen des angenommenen Mechanismus zu der Reaktion, wenn die erste charakteristische Funktion ($F(\omega)$) konstant ist.

**14.** Vorrichtung nach Anspruch 13, **dadurch gekennzeichnet, dass** sie eine Einheit zum Berechnen einer thermokinetischen Konstante auf der Basis repräsentativer Gleichungen dieser Konstante für den durch die Einheit zum Zuordnen (38) zugeordneten Mechanismus aufweist, wobei die repräsentativen Gleichungen von mindestens den gemessenen Amplituden erster Ordnung ($A^{1sin}$, $A^{1cos}$) abhängen.

**Claims**

**1.** A method for determining the reaction mechanism of a reaction, in which at least one first reagent (A) is transformed into at least one first product (C), the process including the following steps:

16

(a1) carrying out a reaction to transform the first reagent (A) into a first product (C) under given experimental conditions, the reaction comprising the periodic variation at a given frequency of a control parameter (T) influencing the reaction;

(a2) scanning a plurality of given frequencies of periodic variations of the control parameter (T) influencing the reaction;

(a3) for each studied frequency, measuring a property representative of the concentration of at least one of the species under the effect of the periodic variation, to determine at least the first-order oscillation amplitudes ($A^{1sin}$, $A^{1cos}$) of the concentration.

**characterized in that** the method further comprises the following steps:

(a4) choosing a reaction presumed reaction mechanism;

(a5) selecting at least one first function ($F(\omega)$) characteristic of a thermokinetic property that is invariant with the oscillation frequency for said supposed reaction mechanism, the first characteristic function ($F(\omega)$) being calculated at least from first-order oscillation amplitudes ($A^{1sin}$, $A^{1cos}$) of the concentration (A) of at least said species;

(a6) calculating a plurality of values of the first characteristic function ($F(\omega)$) from first-order oscillation amplitudes ($A^{1sin}$, $A^{1cos}$) obtained experimentally for the plurality of frequencies of the periodic excitation described in the step (a2);

(a7) analyzing the calculated values of the first characteristic function ($F(\omega)$) to determine whether the first characteristic function ($F(\omega)$) is constant based on the calculated values;

(a8) when the first characteristic function ($F(\omega)$) is constant, assigning the presumed mechanism to said reaction.

**2.** The method according to claim 1, **characterized in that** the control parameter (T) influencing the reaction is the temperature, step (a1) including the periodic variation of the temperature at the given frequency, step (a2) including the scanning of a plurality of temperature variation frequencies.

**3.** The method according to claim 1 or claim 2, **characterized in that** for each frequency, it includes determining second-order oscillation amplitudes ($A^{2sin}$, $A^{2cos}$) of the concentration in at least one of the species, the first characteristic function ($F(\omega)$) selected in step (a5) being calculated based on first-order oscillation amplitudes ($A^{1sin}$, $A^{1cos}$), and based on second-order oscillation amplitudes ($A^{2sin}$, $A^{2cos}$).

**4.** The method according to any one of the preceding claims, **characterized in that** the first characteristic function ($F(\omega)$) is calculated based on a reduced form ($\varepsilon_{\pm1}$) of the ratio of the activation energies to the perfect gas constant multiplied by the mean temperature of the sample.

**5.** The method according to any one of the preceding claims, **characterized in that** it includes selecting at least one second characteristic function ($G(\omega)$); ($H(\omega)$) of a second thermokinetic property that is invariant with the oscillation frequency for said presumed mechanism, the second characteristic function ($G(\omega)$); ($H((\omega)$) being calculated from at least the first-order oscillation amplitudes ($A^{1sin}$, $A^{1cos}$) of the concentration (A) of at least said species, the method including determining a plurality of values of said second characteristic function ($G(\omega)$); ($H(\omega)$) from first-order oscillation amplitude measurements obtained in step (a3) for a plurality of frequencies of the periodic excitation and the analysis of calculated values of the second characteristic function ($G(\omega)$); ($H((\omega)$) to determine whether the second characteristic function ($G(\omega)$); ($H(\omega)$) is constant.

**6.** The method according to any one of the preceding claims, **characterized in that** the second characteristic function ($G(\omega)$) is calculated based on the relaxation time ($\tau_1$) of the presumed reaction mechanism.

**7.** The method according to any one of the preceding claims, **characterized in that** the second characteristic function ($H(\omega)$) is calculated on the basis of the reaction enthalpy ($\Delta H_1$) of the presumed reaction mechanism.

**8.** The method according to any one of the preceding claims, **characterized in that** it includes selecting a non-constant secondary characteristic function ($I(\omega)$) for the presumed reaction mechanism, the secondary characteristic function ($I(\omega)$) having a constant sign irrespective of the oscillation frequency for the presumed reaction mechanism, the method including the determination of a plurality of values of the secondary characteristic function ($I(\omega)$) from first-order oscillation amplitude measurements obtained in step (a3), and analyzing the sign of those values to determine whether the sign is constant.

**9.** The method according to any one of the preceding claims, **characterized in that** the first characteristic function

$(F(\omega))$ is expressed on the basis of at least one parameter chosen from among the stationary term $A^0$ of the concentration variations in a species A, the coefficients $A^{1sin}$, $A^{1cos}$, and optionally the coefficients $A^{2sin}$, $A^{2cos}$ that correspond to the first-order and second-order oscillation amplitudes, respectively, of the concentration of that species A, the pulse $\omega$, and/or the value N of the concentration of that species A before the beginning of the reaction.

10. The method according to any one of the preceding claims, **characterized in that** the presumed mechanism is chosen from among a mechanism of type A + B = C; A + B = 2 C; 2A + B = C; A + B + C = 2C; A + B = C = D = A + B.

11. The method according to any one of the preceding claims, **characterized in that** it includes a step for calculating at least one thermokinetic constant ($\tau_1$, $\Delta_1 H$), based on equations representative of that constant for the mechanism assigned in step (a8), the representative equations depending at least on the measured first-order amplitudes ($A^{1sin}$, $A^{1cos}$).

12. The method according to any one of the preceding claims, **characterized in that** when the first characteristic function ($F(\omega)$) is not constant in step (a7), the method includes the choice of another reaction presumed reaction mechanism and the repetition of steps (a5) to (a8) for the other presumed reaction mechanism.

13. A device (10) for determining the reaction mechanism of a reaction, in which at least one first reagent (A) is transformed into at least one first product (C), including:

- an assembly (12) for carrying out a reaction to transform the first reagent (A) into a first product (C) under given experimental conditions, the carrying out assembly (12) comprising a unit (18) for periodic variation of a control parameter (T) influencing the reaction at a given frequency;
- means (28) for scanning a plurality of given frequencies of periodic variations of the control parameter influencing the reaction;
- a unit (20) for measuring, for each frequency, a property representative of the concentration of at least one of the species (A) under the effect of the periodic variation, capable of determining at least the first-order oscillation amplitudes ($A^{1sin}$, $A^{1cos}$) of the concentration of that species,

**characterized in that** the device (10) further includes an analysis assembly (14) comprising:

- a unit (30) for choosing a reaction presumed reaction mechanism;
- a unit (32) for selecting at least one first function ($F(\omega)$) characteristic of a thermokinetic property that is invariant with the oscillation frequency for said supposed reaction mechanism, the first characteristic function ($F(\omega)$) being calculated at least from first-order oscillation amplitudes ($A^{1sin}$, $A^{1cos}$) of the concentration of at least said species (A);
- a unit (34) for calculating a plurality of values of the first characteristic function ($F(\omega)$) from first-order oscillation amplitude measurements ($A^{1sin}$, $A^{1cos}$) obtained experimentally for the plurality of frequencies of the periodic excitation generated using the unit (28);
- a unit (36) for analyzing calculated values of the first characteristic function ($F(\omega)$) to determine whether the first characteristic function ($F(\omega)$) is constant based on the calculated values;
- a unit (38) for assigning the presumed mechanism to said reaction when the first characteristic function ($F(\omega)$) is constant.

14. The device according to claim 13, **characterized in that** it includes a unit for calculating a thermokinetic constant based on equations representative of that constant for the mechanism assigned by the assigning unit (38), the representative equations depending on at least the measured first-order amplitudes ($A^{1sin}$, $A^{1cos}$).

## FIG.1

## FIG.2

## FIG.8

## FIG.9

FIG.3

FIG.4

**FIG.5**

**FIG.6**

FIG.7

**EP 2 788 115 B1**

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- US 20070043546 A

**Littérature non-brevet citée dans la description**

- **BRAUN et al.** Lock-in by molecular multiplication. *Appl. Phys. Lett.,* 2003, vol. 83, 5554-5556
- Thermal characterisation of a microfluidic cell using the 3 $\Omega$ method. *Digest of the Technical Papers of the 14th International Conference on Solid State Sensors, Actuators and Microsystems,* 2007, U933-U934
- Temperature modulation and quadrature detection for selective titration of two-state exchanging reactants. *Analytical Chemistry,* 2011, 2076-2484